# EUROPÄISCHE PATENTSCHRIFT

(11) **EP 1 615 589 B1**
(45) Veröffentlichungstag und Bekanntmachung des Hinweises auf die Patenterteilung: **30.07.2008**
(21) Anmeldenummer: 04727005.3
(22) Anmeldetag: 13.04.2004
(51) Int. Cl.: A61F 2/00, A61L 31/10, A61L 15/28, A61L 15/64, A61L 31/04

(54) **FLÄCHIGES IMPLANTAT**
PLANAR IMPLANT
IMPLANT PLAT

(30) Priorität: 17.04.2003 DE 10318801
(43) Veröffentlichungstag der Anmeldung: 18.01.2006
(73) Patentinhaber: AESCULAP AG & Co. KG, 78532 Tuttlingen (DE)
(72) Erfinder: ODERMATT, Erich, K., CH-8200 Schaffhausen (CH); ABELE, Wolfgang, 78532 Tuttlingen (DE); WEGMANN, Jürgen, 78222 Stockach (DE)
(74) Vertreter: Patentanwälte Ruff, Wilhelm, Beier, Dauster & Partner
(86) Internationale Anmeldenummer: PCT/EP2004/003849
(87) Internationale Veröffentlichungsnummer: WO 2004/093737

(56) Entgegenhaltungen:
- EP-A- 0 099 758
- EP-A- 1 216 717
- EP-A- 1 216 718
- WO-A-01/56475
- WO-A-02/34304
- GB-A- 1 186 796
- US-B1- 6 264 702

## Beschreibung

Die Erfindung betrifft ein flächiges Implantat.

Der Eingeweidebruch, die sogenannte Hernie, stellt eine häufige Erkrankung dar. Hierbei handelt es sich im allgemeinen um ein Hindurchtreten von Organen oder Organteilen aus der natürlichen Körperhöhle durch eine vorgebildete oder erworbene Lücke. Unter den äußeren Brüchen, bei denen stets der Bruchsack vom Bauchfell umschlossen wird, sind Leisten-, Nabel- und Narbenbrüche die häufigsten Formen. Ursachen für das Auftreten von Hernien sind vor allem Muskel- oder Bindegewebsflächen im Zusammenhang mit Überbelastungen, altersbedingter Erschlaffung, angeborener Schwächung der Bauchdecke oder ungenügende Narbenbildung nach einem Leibesschnitt (Narbenbruch).

Eine effektive Behandlung ist in den meisten Fällen durch einen operativen Eingriff möglich, bei dem der Bruchinhalt aus dem Bruchsack in den Bauch zurückverlagert wird und die Bruchpforte nach vorheriger Implantation eines sogenannten Herniennetzes verschlossen wird. Es sind zahlreiche Herniennetze bekannt. Sie sind in der Regel gewirkte Netze. Sie können aus Polypropylen, PTFE, Polyurethan oder auch Polyester bestehen. Zum Beispiel zeigt die Offenlegungsschrift WO-A-0 234 304 ein solches Herniennetz.

Es gibt aber auch andere flächige Implantate, die zum zumindest vorübergehenden Verbleib im Körper bestimmt sind, hierzu gehören beispielsweise Harninkontinenzbänder und auch sogenannte Patches, mit denen Wundstellen oder Verletzungen bei Organen abgedeckt werden.

Zur Fixierung werden die flächigen Implantate in der Regel an entsprechende Körperstellen angenäht oder sie sind in ihrer Oberfläche so stark strukturiert, dass eine mechanische Verankerung stattfindet. Dies ist beispielsweise bei den Harninkontinenzbändern der Fall, die zum Teil zackige Randbezirke aufweisen, mit denen sie sich in Binde- und Muskelgewebe verankern.

Die Implantate können teilweise oder vollständig resorbierbar sein, indem Fadenmaterial aus resorbierbaren Kunststoffen mit eingearbeitet ist oder die Netze mehrlagig ausgebildet sind, indem ein Netz aus einem nicht resorbierbaren Material mit einem Netz aus resorbierbarem Material kombiniert ist oder die Netze vollständig aus resorbierbarem Material gefertigt sind. Als resorbierbare Materialien kommen vor allem Polymere und Copolymere von Lactid, Glykolid, Trimethylcarbonat, Epsiloncaprolacton, Polyhydroxybuttersäure in Frage.

Die Erfindung liegt die Aufgabe zugrunde, flächige Implantate zu schaffen, die eine einfachere und schonendere Befestigung im Körper ermöglichen.

Diese Aufgabe wird gelöst durch ein flächiges Implantat gemäß Anspruch 1 mit einem flächigen Träger mit zwei Seiten, wobei auf mindestens einer Seite des Trägers eine resorbierbare Haftschicht angeordnet ist, die am menschlichen oder tierischen Gewebe zu haften vermag.

Ein mit einer derartigen Haftschicht versehenes Implantat ist selbsthaftend, so dass eine zusätzliche Fixierung entbehrlich ist. Die resorbierbare Haftschicht gibt eine Vorfixierung des Implantats, bis das Implantat bestimmungsgemäß an- bzw. eingewachsen ist. Die Resorptionszeit des Materials der Haftschicht kann entsprechend eingestellt werden.
Die haftende Schicht gewährleistet eine homogenere Anpassung an das Gewebe. Dadurch erfolgt eine gleichmässigere und vollständigere Durchwachsung mit Bindegewebe.

In der Chirurgie sind verschiedene Kleber bekannt. Beispielhaft seien hier genannt Kleber aus Thrombin und Fibrinogen, wobei Thrombin aktiv in die Blutgerinnungskaskade eingreift. Solche Kleber sind unter dem Gesichtspunkt der BSE- und HIV-Problematik etwas in den Hintergrund geraten. Häufig werden auch Kleber auf der Basis von Cyanacrylat verwendet, diese sind aber kritisch in der Handhabung und auch in Bezug auf die Resorbierbarkeit.

Gemäß der Erfindung wird die Haftschicht im wesentlichen aus mindestens einem, freie Aldehydgruppen tragenden Polymer gebildet, dessen Aldehydgruppen mit nukleophilen Gruppen des Gewebes zu reagieren vermögen. Eine solche Haftschicht bildet eine kovalente Haftverbindung mit den nukleophilen Gruppen des Körpergewebes, insbesondere mit Aminogruppen, SH-Gruppen und OH-Gruppen. Dabei wird eine kovalente Haftverbindung, beispielsweise durch Iminbindungen zwischen den Aldehydgruppen des Polymers und Aminogruppen des Blutes, Serums und vor allem des umgebenden Körpergewebes gebildet. Solche Iminbindungen (Schiff'sche Basen) sind reversible kovalente Bindungen, welche stärker als reine ionische Bindungen sind und ein gutes und gleichmässiges Haften des Implantats am Körpergewebe ermöglichen. Im Falle SH- bzw. OH-Gruppen bildet das erfindungsgemäße Implanat zwischen Haftschicht und Körperteilen Haftverbindungen in Form von Acetal- bzw. Thioacetal-Bindungen aus, die sich entsprechend der Iminbindungen verhalten.

1 bis 5 % aller implantierten Netze zeigen Infektionen durch persistierende Bakterien, welche zu Spätabszessen führen können. Mit der Verwendung von Aldehydgruppen tragenden Polymeren, insbesondere Dextranaldehyd, wird eine desinfiszierende und bakterizide Wirkung auf das Wundgebiet, wie auch auf das Netz ausgeübt, welche zu einer reduzierten Infektionsrate und damit zu einer verminderten kurz- bis langfristigen Rezidivrate führen wird. Die antiinfektive Wirkung kann durch Beimischung von Nano-Silberpartikeln verstärkt werden.

In einer Ausführungsform besteht die Haftschicht nur aus einem Polymer. Bei anderen Ausführungsformen besteht die Haftschicht aus einer Kombination unterschiedlicher Polymere. In weiteren Ausführungsformen verfügt das Polymer der Haftschicht über Vernetzungen, über welche sich die Stabilität und Härte der Haftschicht einstellen lassen. Die Degradationsdauer der Haftschicht lässt sich ebenfalls durch Zusatz von Vernetzern erhöhen. Normalerweise liegt das Polymer in der Haftschicht in unvernetzter Form vor. Es ist weiterhin möglich, Zusatzstoffe wie Weichmacher einzulagern. Ferner pharmakologisch wirksame Substanzen, die aus der Haftschicht an das umgebende Gewebe und an Körperflüssigkeiten abgegeben werden, wie beispielsweise wachstumsförderne Wirkstoffe, wundheilende Wirkstoffe, Desinfektionsmittel, Antibiotika und dergleichen.

Die Haftschicht kann mindestens eine Seite des Implantats auch nur teilweise bedecken. Dies kann dann erwünscht sein, wenn nur einzelne Fixpunkte erforderlich sind. In der Regel ist die Seite des Implantats vollständig von der Haftschicht bedeckt. Eine offene Struktur, insbesondere eine poröse Struktur der Haftschicht ist bevorzugt. Die Haftschicht kann so angebracht sein, dass sie auch nur randständig am Träger vorliegt. Je nach Material und Materialstruktur kann dadurch das Einwachsen eines möglicherweise kritischen Randgebietes des Trägers erleichtert und verstärkt werden.

In der Regel ist nur eine Seite des Trägers mit der Haftschicht versehen.
In besonderen Fällen kann die Haftschicht auf beiden Seiten des Implantats vorgesehen sein. Dies ist dann der Fall, wenn das Implantat zum Verbinden von Körperteilen unter Zwischenlegung des Implantats dient. Die Verbindung von Körperteilen wird bevorzugt mit resorbierbaren flächigen Implantaten durchgeführt, die durch das zusätzlich aufgetragene Polymer, bevorzugt Aldehydgruppen tragender Dextranaldehyd und/oder Aldehydgruppen tragender Polyvinylalkohol, eine Adaptation gewährleisten.

Es ist auch möglich, die Haftschicht auf einer Seite des Implantats vorzusehen und auf der anderen Seite des Implantats eine Antihaftschicht. Dies ist besonders bei Herniennetzen von Vorteil, bei denen eine gute Verbindung mit der Innenseite der Bauchwand erwünscht ist, aber eine Verbindung mit den Organen des Bauchraumes vermieden werden soll. Die Antihaftschicht ist vorzugsweise eine geschlossene Schicht und besitzt insbesondere eine glatte Oberfläche.

Materialien für Antihaftschichten sind bekannt. Hier eignet sich besonders Polyvinylalkohol (PVA), insbesondere mit einem Molekulargewicht von 20.000 bis 200.000. Der Polyvinylalkohol kann insbesondere zur Steuerung seiner Resorptionsdauer in bekannter Weise vernetzt sein.

Die Verwendung von Polyvinylalkohol und seine Verarbeitung als Adhäsionsprophylaxe ist beispielsweise in der WO 02/09789 A2 beschrieben, auf deren Inhalt hier verwiesen wird. Auch Carboxymethylcellulose (CMC), gegebenenfalls in Verbindung mit Polyvinylalkohol eignet sich als Antihaftschicht. Auch die Antihaftschicht kann Wirkstoffe, wie oben erwähnt, enthalten.

Die Haftschicht des erfindungsgemäßen Implantats ist vorzugsweise offenporig und insbesondere saugfähig ausgebildet. Auf diese Weise kann durch Aufnahme von Körperflüssigkeit die Haftung beschleunigt werden. Mit Vorteil ist die Haftschicht hydrophil. Bei einer besonders bevorzugten Ausführungsform vermag die Haftschicht durch Quellung wässrige Flüssigkeiten aufzunehmen, was sich insbesondere in Kombination mit der Saugfähigkeit günstig auswirkt.

In einer besondere Ausführungsform besitzt die Haftschicht eine faserförmige Struktur und liegt vorzugsweise in Form eines insbesondere dreidimensionalen, Vlieses mit einem fasrigen Aufbau vor, welches eine Gesamtoberfläche besitzt, die um ein mehrfaches größer ist als die Außenfläche des Vlieses. Es ist auch denkbar, dass die Haftschicht in Form eines offenporigen flächigen Schaumes vorliegt. Auch hier liegt eine im Verhältnis zur Außenfläche vielfach größere innere Oberfläche vor. Die Haftschicht kann auch in Form einer Folie oder Membran vorliegen. Beispielsweise kann die Haftschicht durch Pressen oder Walzen eines Schaumes oder eines Lyophilisates gebildet sein. Als weitere technische Herstellungsmöglichkeit kommt das direkte Rakeln des Haftvermittlers sowie das Sprayen auf den flächigen Träger in Frage.

Die Haftschicht enthält mit Vorteil wasserlösliche Anteile und ist insbesondere insgesamt wasserlöslich. Dadurch kann die Haftschicht mit fortschreitendem Einwachsen des Implantats durch Auflösen entfernt werden, so dass ein Abbau an Ort und Stelle nicht erforderlich ist.

Auch die Zeit des Auflösens im Körper kann durch chemische und/oder physikalische Maßnahmen, insbesondere durch den Grad einer Vernetzung, durch Gefrier-/Auftauzyklen, durch unterschiedliche Struktur und Domänenbildungen eingestellt werden.

Das Implantat ist bei einer bevorzugten Ausführungsform flexibel ausgebildet. Dies gilt dementsprechend auch für seine Haftschicht. Die Haftschicht braucht nicht vollständig trocken zu sein. Sie kann auch mindestens teilweise als Weichmacher Wasser enthalten oder sogar in Form eines Hydrogels oder Gels vorliegen.

Gemäß einer bevorzugten Ausführungsform handelt es sich bei dem Aldehydgruppen tragenden Polymer um ein oxidiertes, insbesondere bioabsorbierbares Polysaccharid. Als oxidierte Polysaccharide sind Stärke, Agar, Zellulose, Alginsäure, Xanthan und Hyaloronsäure denkbar. In einer besonderen Ausführungsform handelt es sich bei dem Polysaccharid um Dextranpolysaccharid. Das Aldehydgruppen tragende Polymer der Haftschicht kann auch ein synthetisches Polymer sein, insbesondere Polyethylenglykol (PEG), das vorzugsweise verzweigt ist. Bei dieser Ausführungsform besitzt das Polyethylenglykol mindestens drei endständige Aldehydgruppen, welche mit den nukleophilen Gruppen des Körpergewebes kovalente Bindungen auszubilden vermögen. Als weiteres Aldehydgruppen tragendes synthetisches Polymer kommt Polyvinylalkohol (PVA), insbesondere verzweigter Polyvinylalkohol in Frage, der vorzugsweise mindestens drei endständige Aldehydgruppen aufweist.

In weiteren Ausführungsformen können auch andere biokompatible Polyole oder Polyethylenoxid (PEO) als Polymergrundgerüst des Aldehydgruppen tragenden Polymers vorgesehen sein.

Bei dem Polymer der Haftschicht können die Aldehydgruppen innerhalb des Moleküls durch einen Spacer vom Polymergrundgerüst beabstandet angeordnet sein. Dies kann insbesondere bei oxidiertem Polyethylenglykol oder Polyvinylalkohol von Vorteil sein. Die Aldehydgruppen tragenden Polymere können mit keine Aldehydgruppen tragenden Polymeren, wie insbesondere Polyvinylalkohol und/oder Carboxymethylcellulose verstärkt sein.

In der Haftschicht enthaltener Dextranpolyaldehyd besitzt mit Vorteil einen Anteil von zum Aldehyd oxidierten Glukoseeinheiten von mindestens 20%, vorzugsweise 35 bis 100% und insbesondere 50 bis 85%. Durch einen hohen Anteil an zum Aldehyd oxidierten Glukoseeinheiten wird eine Vielzahl von kovalenten Bindungen und dadurch eine starke Haftverbindung zwischen Implantat und Körpergewebe erzielt.

Die Haftschicht des erfindungsgemäßen Implantats kann auf verschiedene Weise mit dem flächigen Träger verbunden werden. So kann die Haftschicht als gesonderte Schicht oder Membran hergestellt werden, die dann mit dem Träger des Implantats verbunden wird. Bevorzugt ist es, die Haftschicht unmittelbar auf dem Träger auszubilden. Die Verbindung der Haftschicht mit dem Träger kann unter Ausnutzung von Klebeeigenschaften der Haftschicht oder gegebenenfalls unter Verwendung eines zusätzlichen insbesondere resorbierbaren Klebers vorgenommen werden. Die Ausbildung der Haftschicht auf dem Träger kann vorgenommen werden, indem das mindestens noch teilweise flüssige oder klebrige Material der Haftschicht mit dem Träger in Verbindung gebracht wird und dann getrocknet wird. So kann der Träger beispielsweise in eine Lösung des Polymers der Haftschicht eingetaucht oder mit dieser beschichtet und die Haftschicht dann durch Lufttrocknung oder Lyophilisation ausgebildet werden. Es ist auch möglich, das Material der Haftschicht in Form einer Sprühschicht, insbesondere eines Sprühvlieses auf den Träger aufzutragen. Auch andere bekannte Beschichtungsmöglichkeiten sind möglich. Eine Vorfertigung der Haftschicht kann durch bloßes Trocknen von Lösungen vorgenommen werden. Poröse Haftschichten werden insbesondere durch Lyophilisation von Lösungen erhalten, wobei eine sehr luftige Struktur erhältlich ist, wenn die Lösungen vor der Lyophilisation aufgeschäumt oder größere Hohlräume durch Zugabe von zerstoßenem Eis vor der Gefriertrocknung der Lösung geschaffen werden. Einseitige Haftschichten können durch Einpressen des flächigen Trägers auf eine lyophilisierte Struktur geschaffen werden. Sandwich- oder beidseitige Haftschichtstrukturen können durch Einpressen des flächigen Trägers zwischen zwei z.B. lyophilisierte Strukturen erhalten werden.

Bei bevorzugten Ausführungsformen kann die Haftschicht aufgrund ihrer schwammartigen Struktur und Porosität und ihres hydrophilen Charakters mindestens das 30-fache ihres Eigengewichtes an Flüssigkeit aufnehmen. Außerdem ist die Haftschicht in der Lage mindestens das 4-fache ihres Eigengewichts an Hämoglobin aufzunehmen. Dadurch wird neben einer guten Haftverbindung gleichzeitig eine Blutstillung erzielt, wenn dies erwünscht ist.

Das mindestens eine die Aldehydgruppen tragende Polymer kann vor der Herstellung der Haftschicht mit einem Vernetzungsmittel vernetzt sein. Als Vernetzungsmittel kommen bifunktionelle Amine, insbesondere Diaminosäuren Lysin, Ornitin, Arginin oder Triethylenglykoldiamin, ferner multifunktionelle Amine, insbesondere die Polyaminosäure Polylysin, bi- bzw. multifunktionelle SH- oder NH₂-Gruppen haltige Moleküle, insbesondere Cystein oder Polycystein, oder bi- bzw. multifunktionelle Thiole bzw. Peptide in Frage. Besonders bevorzugt ist Chitosan.

In einer besonderen Ausführungsform besitzt die Haftschicht eine mindestens einseitig strukturierte Oberfläche. Die strukturierte Oberfläche verbessert die Haftung der Haftschicht auf dem Gewebe. Es sind verschiede Arten der Strukturierung denkar, wie eine quadratische, zackige, geflochtene, gewebte oder spiralförmige Struktur. Durch die Strukturierung wird die mechanische Reibung zwischen Gewebe und der Haftschicht erhöht und das Implantat hält nach der Applikation besser an der angebrachten Position. Die Strukturierung kann bevorzugt auch durch die Grundstruktur des Trägers, z.B. des Gewirks, gebildet sein. So können Fasern einer textilen Struktur des Trägers mit die Haftschicht bildendem Material überzogen sein. Hierbei ist es bevorzugt, dass die offene bzw. offenporige Struktur des textilen Trägers erhalten bleibt. Es reicht aus, wenn die einzelnen Fasern mit Haftschichtmaterial überzogen sind.

Die Strukurierung kann auch durch entsprechend strukturierte Lyophilisationsschalen oder durch ein Prägen im Anschluss an die Herstellung des Implantats oder der Haftschicht erzeugt werden.

Der flächige Träger für die Haftschicht ist wie bereits erwähnt, vorzugsweise flexibel ausgebildet. Er besitzt vorzugsweise auf mindestens einer Seite eine offene Struktur, die zum Einwachsen von Zellgewebe geeignet ist. Hierzu eignen sich insbesondere textile Materialien, wobei Gewebe, Geflechte, Gestricke und insbesondere Gewirke bevorzugt sind. Diese können aus monofilen Fäden und/oder multifilen Fäden, welche resorbierbar und/oder nicht resorbierbar sind, gefertigt sein. Sofern das Implantat auf Dauer im Körper verbleiben soll, ist der Träger mindestens zum Teil aus nicht resorbierbarem Material gefertigt und zwar derart, dass seine Funktion erhalten bleibt. So kann der Träger beispielsweise teilweise resorbierbar sein, wenn eine gewisse Anfangsstabilität erforderlich ist, die dann aber mit der Zeit nicht mehr notwendig ist, beispielsweise wenn das Körpergewebe durch den Heilungsprozess in der Lage ist, die Funktion des Trägers mindestens teilweise zu übernehmen. Ist eine Langzeitwirkung des Implantats nicht erforderlich oder nicht erwünscht, kann das Implantat insgesamt vorzugsweise resorbierbar sein, so dass es mit der Zeit verschwindet, wenn es seine Funktion erfüllt hat.

Das erfindungsgemäße Implantat kann aufgrund der unterschiedlichen Ausbildung seiner Oberflächen vielseitig in der Chirurgie eingesetzt werden. Es kann dazu verwendet werden, bestimmte Organe abzudecken oder Gewebeteile miteinander zu verbinden. Es kann, wenn eine Seite eine Antihaftschicht aufweist, auch dazu verwendet werden, ein unerwünschtes Zusammenwachsen von Körperteilen zu verhindern.

Das erfindungsgemäße Implantat kann auch in unterschiedlicher Gestalt vorliegen. In der Regel liegt es in Form eines flexiblen Flächenmaterials vor. Es kann aber auch dreidimensional geformt sein, insbesondere rohrförmig mit einer Außenseite und einer Innenseite. Es kann auch die Gestalt eines Ringes besitzen. Insbesondere dann, wenn das Implantat eine dreidimensionale Struktur besitzt, kann es auch formstabil, insbesondere elastisch formstabil sein.

Solche relativ formstabilen Implantate können insbesondere als Verbindungsstücke oder Versteifungsstücke für rohrförmige Hohlorgane ausgebildet sein wie Gefäße oder Darmabschnitte. Es ist auch denkbar, die Oberfläche von Vollmaterialien oder linearen Materialien, wie von chirurgischem Nahtmaterial oder von chirurgischen Klammern, mit einer entsprechenden Haftschicht zu versehen, um deren Verankerung, Einwachsverhalten und Infektionsprophylaxe im Körpergewebe zu verbessern.

Das erfindungsgemäße Implantat lässt sich gut sterilisieren und liegt im Gebrauchszustand in steriler Form vor, insbesondere in einer sterilen Verpackung, die erst kurz vor der Implantation geöffnet wird.

Weitere Merkmale der Erfindung ergeben sich durch die nachfolgende Beschreibung von bevorzugten Ausführungsformen und Beispielen in Verwendung mit den Unteransprüchen. Hierbei können die einzelnen Merkmale der Erfindung allein oder in Kombination miteinander verwirklicht sein. Die beschriebenen Ausführungsformen dienen zur Erläuterung und zum besseren Verständnis der Erfindung und sind nicht einschränkend zu verstehen.

### Beispiele

### Beispiel 1

### Herstellung eines flächigen Implantats mit beidseitiger Haftschicht

Dextranaldehyd wird in bidestilliertem Wasser bei 50°C gelöst. In eine flache Schale wird so viel von der Lösung gegossen, dass der Boden gerade bedeckt ist. Etwa zu viel eingegossene Lösung wird wieder ausgegossen. Auf die Lösung wird ein Herniennetz vorsichtig aufgelegt. Die Lösung wird daraufhin lyophilisiert, wobei sich auf dem Herniennetz auf einer Seite eine Haftschicht aus Dextranaldehyd ausbildet. Die Dicke der Haftschicht entspricht der Füllhöhe der Dextranaldehydlösung vor der Lyophilisation. Die Haftschicht hat die Struktur eines Vlieses. Es können Haftschichten unterschiedlicher Dichte und Festigkeit aus Lösungen mit unterschiedlicher Konzentration an Dextranaldehyd hergestellt werden, wobei sich mit 1%igen Dextranaldehydlösungen und Dextranaldehydlösungen mit höherer Konzentration im wesentlichen geschlossene Haftschichten herstellen lassen, die mit zunehmender Konzentration an Festigkeit zunehmen.

Bei einem solchen Herniennetz wird nur die eine Seite des Netzes durch die Adhäsionskraft der Oberfläche festgehalten. Dies kann noch dadurch verstärkt werden, dass die von der Haftschicht abweisende Seite des Herniennetzes zur Erzielung einer Antihaftschicht mit einer viskosen Lösung von Polyvinylalkohol besprüht wird, worauf im Luftstrom getrocknet wird. Es wird somit ein Herniennetz erhalten, das mit der Seite, die zur Bauchwand hingerichtet ist, mit dieser eine schnelle und gute Haftverbindung eingeht, so dass sich ein Fixieren des Herniennetzes durch Annähen oder Anklammern erübrigt. Die zur Bauchinnenseite weisende Seite des Herniennetzes besitzt aufgrund der Antiadhäsionsschicht eine Prophylaxe gegen ein unerwünschtes Anwachsen von Organen des Bauchraums, zumindest bis die Wundheilung abgeschlossen ist.

### Beispiel 2

Der erste Schritt von Beispiel 1 wird wiederholt, wobei jedoch so viel Dextranaldehyd in die Schale gegossen wird, dass das Herniennetz nach dem Einlegen beidseitig von der Lösung benetzt ist. Durch Lyophilisation wird ein Netz erhalten, das auf beiden Seiten eine Haftschicht aus Dextranaldehyd besitzt. Durch dieses Implantat ist es möglich, Flächen von Körpergewebe miteinander zu verbinden. Besteht das Netz aus resorbierbarem Fadenmaterial, dann verschwindet das Implantat nach Zusammenwachsen der Gewebeteile.

### Beispiel 3

Ein formstabiler aber noch elastischer Rohrabschnitt aus resorbierbarem bioverträglichem Kunststoff wie PGA, ein Terpolymer aus Lactid (65) TMC (19) und Caprolacton (16), ein Copolymer aus L-Lactid (86) und TMC (14) und/oder Polyglykolidlactid (90/10), der die Struktur eines elastischen Rohrgeflechts besitzt, wird vollständig in eine 5%ige Dextranaldehydlösung getaucht, wonach man die Lösung im Luftstrom trocknen lässt. Dabei wird ein durchmesserstabiler Verbindungsring erhalten, der sich zur gegenseitigen Verklebung von Darmenden nach einer Teilresektion eignet.

### Beispiel 4

### Tauchverfahren

Premilene^{®}-Netze (gewirkte Netze aus monofilen Polypropylenfäden) wurden drei Tauchvorgängen in unterschiedlich konzentrierten Lösungen von Dextranaldehyd (DA) unterworfen. Hierzu wurden die Netze für 60 Sekunden in die jeweilige Lösung getaucht und anschließend an der Luft bis zur Gewichtskonstanz getrocknet. Die Ergebnisse sind in Tabelle 1 zusammengefasst.

**Tabelle 1: Beschichtung von Premilene-Netzen mit unterschiedlich konzentrierten DA-Lösungen (Tauchverfahren). [Gewichtsprozent auf das Gewicht des unbeschichteten Netz bezogen]**

| | | 1. Tauchvorgang | | 2. Tauchvorgang | | 3. Tauchvorgang | |
|---|---|---|---|---|---|---|---|
| DA-Lösung [%] | Gewicht unbeschich-tet [mg] | Gewicht [mg] | Gewichtsprozent [%] | Gewicht [mg] | Gewichtsprozent [%] | Gewicht [mg ] | Gewichtsprozent [%] |
| 2,5 | 125 | 126,9 | 101,5 | 126,9 | 101,5 | 126,4 | 101,1 |
| 5 | 125,9 | 130 | 103,20 | 130 | 103,3 | 129,9 | 103,2 |
| 7,5 | 126,7 | 137,6 | 108,6 | 142 | 112,1 | 151 | 119,2 |
| 10 | 125,2 | 136,9 | 109,3 | 138,5 | 110,6 | 138,5 | 110,6 |

Während mit den niedrig konzentrierten Lösungen nur eine geringe Gewichtszunahme erreicht wurde, war die Belegung mit der 7,5 %igen DA-Lösung sehr hoch. Mit der noch höher konzentrierten 10 %igen DA-Lösung wurde die Beschichtung nicht weiter verbessert.

### Beispiel 5

### Sprühverfahren:

Die Netze wurden insgesamt dreimal mit DA-Lösungen unterschiedlicher Konzentration besprüht und anschließend bis zur Gewichtskonstanz getrocknet. Als Sprüh-Device diente ein Spray Set der Fa. Confluent Surgical. Der Abstand zwischen Düse und Netz betrug 20 cm.

**Tabelle 2: Beschichtung von Premilene-Netzen mit unterschiedlich konzentrierten DA-Lösungen (Sprühverfahren). [Gewichtsprozent auf das Gewicht des unbeschichteten Netz bezogen].**

| | | 1. Sprühvorgang | | 2. Sprühvorgang | | 3. Sprühvorgang | |
|---|---|---|---|---|---|---|---|
| DA-Lösung [% w/v] | Gewicht unbeschichtet [mg] | Gewicht [mg] | Gewichtsprozent [%] | Gewicht [mg] | Gewichtsprozent [%] | Gewicht [mg] | Gewichtsprozent [%] |
| 2,5 | 118,4 | 122,2 | 103,2 | 123,6 | 104,4 | 123,6 | 104,4 |
| 5 | 123,1 | 125,1 | 101,6 | 126,5 | 102,8 | 127,2 | 103,3 |
| 7,5 | 117,3 | 135,4 | 115,5 | 146,8 | 125,1 | 152,8 | 130,3 |
| 10 | 121,8 | 133,1 | 109,3 | 137,7 | 113,1 | 143,3 | 117,7 |

Mit dem Sprühverfahren konnte im Vergleich zum Tauchverfahren ein höherer Beschichtungsgrad erreicht werden. Analog zum Tauchverfahren wurde mit der 7,5%igen DA-Lösung die höchste Gewichtszunahme beobachtet. Bei allen Netzen bleibt die netzartige Struktur erhalten. Die Poren werden durch das Dextranaldehyd nicht verschlossen.

### Beispiel 5a

Unterschiedliche Haftungsschichten auf Netzen können wie folgt hergestellt werden:
1. Ein lyophilisierter Schaum mit einer Dicke von 6mm wird hergestellt
2. Eine Fläche von 10 x 15 cm² wird zurechtgeschnitten
3. Die entsprechende Netzfläche aus Polypropylen wird zurechtgeschnitten und mit bidestilliertem Wasser besprüht
4. Das feuchte Netz wird unter leichtem Druck auf das Lyophilisat gedrückt und angetrocknet.
5. Nach der Trocknung wird die freie Netzseite nochmals besprüht und der entsprechend zugeschnittene PVA-Prophylaxefilm angedrückt und damit befestigt.

### Beispiele 6 und 7

### Safil^{®} Netz (Gewirk aus monofilen und multifilen Fäden aus Polyglykolsäure):

Die Beschichtung der Safil-Netze erfolgte mit einer 10%igen DA-Lösung. Auch hier wurde sowohl das Tauchverfahren als auch das Sprühverfahren angewendet. Die Ergebnisse sind in Tabelle 3 und 4 zusammengefasst. Werden dreidimensionale aufgefaltete Netzstrukturen rotierend von außen besprüht, erhält man ein noch elastisches Netzwerk mit DA hauptsächlich auf der Außenseite.

**Tabelle 3: Beschichtung eines Safil-Netzes mit einer 10%igen DA-Lösung (Tauchverfahren). [Gewichtsprozent auf das Gewicht des unbeschichteten Netz bezogen].**

| | | 1. Tauchvorgang | | 2. Tauchvorgang | | 3. Tauchvorgang | |
|---|---|---|---|---|---|---|---|
| DA-Lösung [%] | Gewicht unbeschichtet [mg] | Gewicht [mg] | Gewichtsprozent [%] | Gewicht [mg] | Gewichtsprozent [%] | Gewicht [mg] | Gewichtsprozent [%] |
| 10 | 92,8 | 107,3 | 115,6 | 107,3 | 115,6 | 111 | 119,6 |

**Tabelle 4: Beschichtung eines Safil-Netzes mit einer 10%igen DA-Lösung (Sprühverfahren). [Gewichtsprozent auf das Gewicht des unbeschichteten Netz bezogen].**

| | | 1. Sprühvorgang | | 2. Sprühvorgang | | 3. Sprühvorgang | |
|---|---|---|---|---|---|---|---|
| DA-Lösung [%] | Gewicht unbeschichtet [mg] | Gewicht [mg] | Gewichtsprozent [%] | Gewicht [mg] | Gewichtsprozent [%] | Gewicht [mg] | Gewichtsprozent [%] |
| 10 | 85,3 | 101,1 | 118,5 | 109,4 | 128,3 | 116,1 | 136,1 |

Wiederum konnte mit dem Sprühverfahren eine größere Gewichtszunahme im Vergleich zum Tauchverfahren beobachtet werden. Im Vergleich zu den Premilene Netzen ist bei den Safil-Netzen die prozentuale Gewichtszunahme höher, d.h. bei Safil ist ein höherer Beschichtungsgrad der Netze möglich.

Es zeigt sich, dass die Poren der Netze durch die Beschichtung nicht verschlossen werden. Ein Verschluss der Poren kann durch Steigerung der Sprüh-/Trockenzyklen erreicht werden.

In der Zeichnung zeigen
- Figur 1:: einen Teilquerschnitt durch ein Herniennetz mit einer Haftschicht und einer Antihaftschicht nach Beispiel 1
- Figur 2:: einen Längsschnitt durch eine Verbindung von Darmendstücken nach einer Teilresektion
- Figur 3:: eine Ansicht der Ausführungsform nach Figur 2.

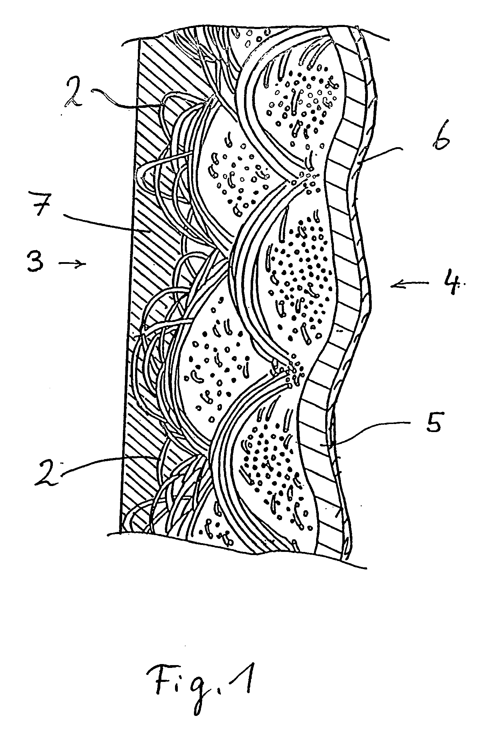

Bei der Ausführungsform nach Figur 1 ist ein textiles Flächengebilde 1 als Kettengewirk aus multifilem Polyethylenterephtalatgarn in Form eines Einfachvelour ausgebildet. Velourschlingen 2 sind aus texturiertem Garn gebildet. Das Gewirk ist porös und flexibel und dient als Träger des erfindungsgemäßen Implantats. Das Gewirk als solches besitzt auf der Velourseite 3 eine offene dreidimensional strukturierte Oberfläche, die aufgrund der Velourschlingen und der Texturierung der Fasern zahlreiche im wesentlichen gleichmäßig über die Oberfläche verteilte Hintergreifmöglichkeiten zum Einwachsen von Körperzellen aufweist. Dabei sind die Öffnungen zwischen den Garnschlingen bzw. den einzelnen Fasern groß im Verhältnis zur Größe von Körperzellen. Dies ermöglicht das Einwachsen eines zusammenhängenden Zellverbundes.

Entsprechend der in Beispiel 1 beschriebenen Verfahrensweise befindet sich auf der Velourseite 3 des Wirkstoffes eine Haftschicht 7 in Form von lyophilisiertem Dextranaldehyd. Da die Dextranaldehydlösung vor der Lyophilisation in die Oberfläche des Gewirks eingedrungen ist, liegt eine im wesentlichen geschlossene aber poröse Haftschicht vor.

Die gegenüberliegende Seite 4 des Gewirks ist aufgrund der Bindung des Gewirks dichter und eher eben. Zusätzlich zu der in Beispiel 1 beschriebenen Verfahrensweise, kann das Gewirk 1 an dieser Seite eine Sprühbeschichtung 5 aus unvernetztem Polyurethan aufweisen, die mit den an der Oberfläche freiliegenden Fasern des Gewirks 1 verbunden ist und die textile Struktur an dieser Oberfläche im wesentlichen verschließt. Die Sprühbeschichtung hat die Struktur eines Sprühvlieses. Die Dicke dieses Sprühvlieses liegt in der Größenordnung von etwa 1/10 bis 1/20 der Gesamtdicke von textilem Flächengebilde und Versiegelungsschicht. Auf der Außenseite der Versiegelungsschicht 5 befindet sich die in Beispiel 1 erwähnte durch Aufsprühen von Polyvinylalkohollösung erzeugte Antiadhäsionsprophylaxe, die die Poren des Sprühvlieses abdichtend verschließt und ein Einwachsen von Zellen während der Wundheilungsphase verhindert. Wird keine Versiegelungsschicht, z.B. aus Polyurethan, benötigt, kann die adhäsionsprophylaktische Schicht aus PVA (6) direkt auf das Gewirk aufgetragen werden.

Die dargestellte Ausführungsform kann als Herniennetz mit guten Hafteigenschaften auf der einen Seite und Antihafteigenschaften auf der anderen Seite eingesetzt werden, wobei eine schnelle und gute Verbindung des Netzes mit der Bauchwand erreicht und ein unerwünschtes Anwachsen von Organen des Bauchraumes verhindert wird.

Je nach den Anwendungserfordernissen kann das Herniennetz aus monofilen oder multifilen Fäden bestehen. Die Netzstruktur kann dünn und leichtgewichtig sein, da die für das Ausbreiten des Netzes erforderlichen Eigenschaften dem Netz durch die Beschichtungen verliehen werden können. Die Netzstruktur kann auch ganz oder mindestens teilweise aus resorbierbaren Materialien bestehen.

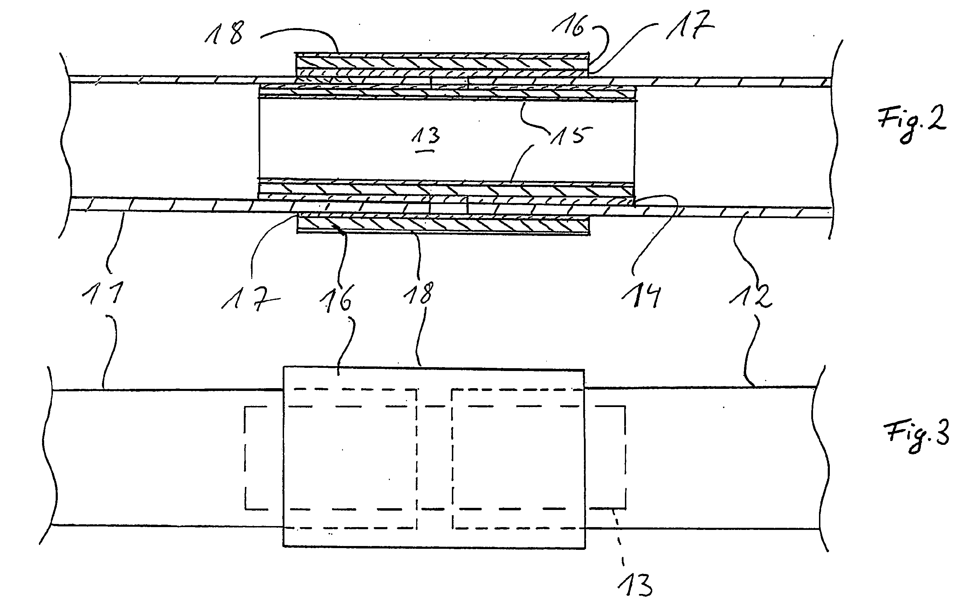

Die Figuren 2 und 3 zeigen in schematischer Darstellung die Verbindung zweier Darmabschnitte nach einer Teilresektion. Die freien Enden der Darmabschnitte 11 und 12 sind über ein rohrförmiges Implantat 13 geschoben, das im wesentlichen wie in Beispiel 3 beschrieben, hergestellt ist. Das Geflecht des Rohrabschnittes ist durch das die Haftschicht bildende Polymer versteift. Bei der hier beschriebenen Ausführungsform dient lediglich die Beschichtung 14 auf der Außenseite des Innenrohres 13 als Haftschicht zur Verbindung mit der Innenseite der Darmwand. Die entsprechende Beschichtung 15 auf der Innenseite hat keine Funktion und wird durch den Darminhalt aufgelöst. Über die Darmverbindung geschoben ist als Außenrohr ein weiterer Rohrabschnitt 16, der eine Haftschicht 17 nur auf seiner Innenseite besitzt, wogegen die Außenseite mit einer Antihaftschicht 18 aus Polyvinylalkohol überzogen ist. Durch das Innenrohr 13 und das Außenrohr 16 werden die Darmenden sowohl von außen als auch von innen mit den entsprechenden rohrförmigen Implantaten verklebt. Die Antihaftschicht 18 verhindert, dass sich der hier befindliche Darmabschnitt mit einem weiteren Darmabschnitt oder einem anderen Organ des Bauchraumes verbindet.

Gleichzeitig mit dem Zusammenwachsen der Darmabschnitte findet eine Auflösung und Resorption sowohl der Haftschicht als auch der Antihaftschicht der Rohrabschnitte statt. Auch das im Inneren des Geflechtes befindliche die Haftschicht bildende Polymer aus Dextranaldehyd wird mit der Zeit aufgelöst, so dass die Flexibilität der Rohrabschnitte aus den Geflechten entsprechend zunimmt. Da die Geflechte aus resorbierbaren Kunststoffen, z.B. ein Terpolymer aus Lactid, TMC und Caprolacton (65/19/16) oder ein Copolymer aus Lactid (86) und TMC (14), bestehen, lösen sich auch die Geflechtsabschnitte als textile Träger des Implantats mit der Zeit auf, so dass letztlich nur noch der wieder zusammengewachsene Darm übrigbleibt.

## Patentansprüche

1. Flächiges Implantat mit einem flächigen Träger (1, 13, 16) mit zwei Seiten, wobei auf mindestens einer Seite des Trägers eine resorbierbare Haftschicht (7, 14, 17) vorgesehen ist, die an menschlichem und tierischem Gewebe zu haften vermag, **dadurch gekennzeichnet, dass** die Haftschicht im wesentlichen von mindestens einem, freie Aldehydgruppen tragendem Polymer, dessen Aldehydgruppen mit nukleophilen Gruppen des Gewebes unter Ausbildung von kovalenten Bindungen zu reagieren vermögen, gebildet ist.

2. Implantat nach Anspruch 1, **dadurch gekennzeichnet, dass** die Haftschicht (7, 14, 17) anti-infektive Eigenschaften aufweist.

3. Implantat nach Anspruch 1 oder 2, **dadurch gekennzeichnet, dass** die Haftschicht die mindestens eine Seite des Trägers mindestens teilweise, vorzugsweise vollständig, bedeckt.

4. Implantat nach einem der Ansprüche 1 bis 3, **dadurch gekennzeichnet**, das die Haftschicht den flächigen Träger lediglich randständig bedeckend und/oder den flächigen Träger randständig überragend ausgebildet ist.

5. Implantat nach einem der vorhergehenden Ansprüche, **dadurch gekennzeichnet, dass** die Haftschicht auf beiden Seiten des Trägers vorgesehen ist.

6. Implantat nach einem der Ansprüche 1 bis 4, **dadurch gekennzeichnet, dass** der Träger auf einer Seite eine Haftschicht und vorzugsweise auf der anderen Seite eine Antihaftschicht aufweist.

7. Implantat nach Anspruch 6, **dadurch gekennzeichnet, dass** die Antihaftschicht eine geschlossene und insbesondere glatte Oberfläche besitzt.

8. Implantat nach einem der vorhergehenden Ansprüche, **dadurch gekennzeichnet, dass** die Haftschicht als offene Schicht und insbesondere saugfähig ausgebildet ist.

9. Implantat nach einem der vorhergehenden Ansprüche, **dadurch gekennzeichnet, dass** die Haftschicht hydrophil ist, insbesondere wässrige Flüssigkeiten durch Quellung aufzunehmen vermag.

10. Implantat nach einem der vorhergehenden Ansprüche, **dadurch gekennzeichnet, dass** die Haftschicht in Form eines, insbesondere dreidimensionalen, Vlieses vorliegt.

11. Implantat nach einem der Ansprüche 1 bis 9, **dadurch gekennzeichnet, dass** die Haftschicht in Form eines offenporigen Schaumes vorliegt.

12. Implantat nach einem der Ansprüche 2 bis 11, **dadurch gekennzeichnet, dass** das Aldehydgruppen tragende Polymer wasserlöslich ist.

13. Implantat nach einem der Ansprüche 2 bis 12, **dadurch gekennzeichnet, dass** es sich bei dem Aldehydgruppen tragenden Polymer um ein oxidiertes, insbesondere bioabsorbierbares, Polysaccharid handelt.

14. Implantat nach Anspruch 13, **dadurch gekennzeichnet, dass** es sich bei dem oxidiertem Polysaccharid um eines aus der Gruppe bestehend aus Stärke, Zellulose, Agar, Dextranaldehyd, Hyaloronsäure, Alginsäure, Chondroitinsulfat und vorzugsweise um Dextranpolyaldehyd, handelt.

15. Implantat nach Anspruch 14, **dadurch gekennzeichnet, dass** der Anteil an zum Aldehyd oxidierten Glukoseeinheiten im Dextranpolyaldehyd mindestens 20%, vorzugsweise 35 bis 100%, insbesondere 50 bis 85%, beträgt.

16. Implantat nach einem der Ansprüche 2 bis 12, **dadurch gekennzeichnet, dass** es sich bei dem Aldehydgruppen tragenden Polymer um ein, insbesondere verzweigtes, Polyethylenglykol mit mindestens drei endständigen Aldehydgruppen handelt.

17. Implantat nach einem der Ansprüche 2 bis 12, **dadurch gekennzeichnet, dass** es sich bei dem Aldehydgruppen tragenden Polymer um einen, insbesondere verzweigten, Polyvinylalkohol mit mindestens drei endständigen Aldehydgruppen handelt.

18. Implantat nach einem der Ansprüche 2 bis 17, **dadurch gekennzeichnet, dass** das mindestens eine Aldehydgruppen tragende Polymer teilweise vernetzt ist.

19. Implantat nach einem der vorhergehenden Ansprüche, **dadurch gekennzeichnet, dass** die Haftschicht an ihrer Außenseite eine strukturierte Oberfläche aufweist.

20. Implantat nach einem der vorhergehenden Ansprüche, **dadurch gekennzeichnet, dass** der flächige Träger porös und flexibel ist, insbesondere von einem textilen Material gebildet wird.

21. Implantat nach einem der vorhergehenden Ansprüche, **dadurch gekennzeichnet, dass** der Träger, insbesondere der textile Träger, mindestens teilweise, insbesondere vollständig, resorbierbar ist.

22. Implantat nach einem der vorhergehenden Ansprüche, **dadurch gekennzeichnet, dass** eine Seite des Trägers mit mindestens einer Antihaftschicht versehen ist, die vorzugsweise resorbierbar ist.

23. Implantat nach Anspruch 21, **dadurch gekennzeichnet, dass** die Antihaftschicht Polyvinylalkohol und/oder Carboxymethylcellulose enthält, insbesondere aus Polyvinylalkohol besteht.

24. Implantat nach einem der vorhergehenden Ansprüche, **dadurch gekennzeichnet, dass** es als Herniennetz ausgebildet ist, das die Haftschicht auf der Seite aufweist, die zur Anlage an die Bauchwand bestimmt ist, und dass vorzugsweise die andere Seite des Herniennetzes mindestens eine Schicht aufweist, die als Antihaftschicht ausgebildet ist und eine Adhäsion von Körpergewebe am Netz verhindert.

25. Implantat nach einem der Ansprüche 1 bis 22, **dadurch gekennzeichnet, dass** es als Pflaster (Patch) ausgebildet ist, das auf mindestens einer Seite die Haftschicht aufweist.

26. Implantat nach einem der Ansprüche 1 bis 22, **dadurch gekennzeichnet, dass** es als Rohrabschnitt vorliegt, der zum Verbinden von rohr- bzw. schlauchartigen Hohlorganen ausgebildet ist.

## Claims

1. Planar implant comprising a planar support (1, 13, 16) with two faces, at least one face of the support being provided with an absorbable adhesive layer (7, 14, 17) which is able to adhere to human or animal tissue, **characterized in that** the adhesive layer is essentially formed from at least one polymer which carries free aldehyde groups and whose aldehyde groups are able to react with nucleophilic groups of the tissue, forming covalent bonds.

2. Implant according to Claim 1, **characterized in that** the adhesive layer (7, 14, 17) has anti-infective properties.

3. Implant according to Claim 1 or 2, **characterized in that** the adhesive layer at least partially covers, preferably completely covers, the at least one face of the support.

4. Implant according to one of Claims 1 to 3,
**characterized in that** the adhesive layer is designed to cover the planar support only around the edges and/or to protrude beyond the edges of the planar support.

5. Implant according to one of the preceding claims, **characterized in that** the adhesive layer is provided on both faces of the support.

6. Implant according to one of Claims 1 to 4, **characterized in that** the support has an adhesive layer on one face and preferably has an anti-adhesive layer on the other face.

7. Implant according to Claim 6, **characterized in that** the anti-adhesive layer has a closed and in particular smooth surface.

8. Implant according to one of the preceding Claims, **characterized in that** the adhesive layer is designed as an open layer and is in particular absorbent.

9. Implant according to one of the preceding claims, **characterized in that** the adhesive layer is hydrophilic and in particular is able to take up aqueous fluids by swelling.

10. Implant according to one of the preceding claims, **characterized in that** the adhesive layer is present in the form of a nonwoven, in particular a three-dimensional nonwoven.

11. Implant according to one of Claims 1 to 9, **characterized in that** the adhesive layer is present in the form of an open-cell foam.

12. Implant according to one of Claims 2 to 11, **characterized in that** the polymer carrying aldehyde groups is soluble in water.

13. Implant according to one of Claims 2 to 12, **characterized in that** the polymer carrying aldehyde groups is an oxidized, in particular bioabsorbable polysaccharide.

14. Implant according to Claim 13, **characterized in that** the oxidized polysaccharide is one from the group comprising starch, cellulose, agar, dextran aldehyde, hyaluronic acid, alginic acid, chondroitin sulfate, and preferably dextran polyaldehyde.

15. Implant according to Claim 14, **characterized in that** the proportion of glucose units oxidized to the aldehyde in the dextran polyaldehyde is at least 20%, preferably 35 to 100%, in particular 50 to 85%.

16. Implant according to one of Claims 2 to 12, **characterized in that** the polymer carrying aldehyde groups is an in particular branched polyethylene glycol with at least three terminal aldehyde groups.

17. Implant according to one of Claims 2 to 12, **characterized in that** the polymer carrying aldehyde groups is an in particular branched polyvinyl alcohol with at least three terminal aldehyde groups.

18. Implant according to one of Claims 2 to 17, **characterized in that** the at least one polymer carrying aldehyde groups is partially crosslinked.

19. Implant according to one of the preceding claims, **characterized in that** the adhesive layer has a structured surface on its outer face.

20. Implant according to one of the preceding claims, **characterized in that** the planar support is porous and flexible, and in particular is formed from a textile material.

21. Implant according to one of the preceding claims, **characterized in that** the support, in particular the textile support, is at least partially absorbable, in particular completely absorbable.

22. Implant according to one of the preceding claims, **characterized in that** one face of the support is provided with at least one anti-adhesive layer which is preferably absorbable.

23. Implant according to Claim 21, **characterized in that** the anti-adhesive layer contains polyvinyl alcohol and/or carboxymethylcellulose, and in particular consists of polyvinyl alcohol.

24. Implant according to one of the preceding claims, **characterized in that** it is designed as a hernia mesh having the adhesive layer on the face which is intended to bear on the abdominal wall, and **in that** the other face of the hernia mesh preferably has at least one layer which is designed as an anti-adhesive layer and prevents adhesion of body tissue to the mesh.

25. Implant according to one of Claims 1 to 22, **characterized in that** it is designed as a patch which has the adhesive layer on at least one face.

26. Implant according to one of Claims 1 to 22, **characterized in that** it is present as a tube section which is designed for connection of tubular hollow organs.

## Revendications

1. Implant plat présentant un support plat (1, 13, 16) avec deux faces, où au moins une face du support est pourvue d'une couche adhésive résorbable (7, 14, 17), qui est capable d'adhérer à des tissus humains et animaux, **caractérisé en ce que** la couche adhésive est essentiellement formée d'au moins un polymère portant des groupes aldéhyde libres, dont les groupes aldéhyde sont capables de réagir avec des groupes nucléophiles du tissu en formant des liaisons covalentes.

2. Implant selon la revendication 1, **caractérisé en ce que** la couche adhésive (7, 14, 17) présente des propriétés anti-infectieuses.

3. Implant selon la revendication 1 ou 2, **caractérisé en ce que** la couche adhésive recouvre au moins partiellement, de préférence totalement l'au moins une face du support.

4. Implant selon l'une quelconque des revendications 1 à 3, **caractérisé en ce que** la couche adhésive est conçue pour recouvrir le support plat seulement autour des bords et/ou pour dépasser des bords du support plat.

5. Implant selon l'une quelconque des revendications précédentes, **caractérisé en ce que** la couche adhésive est prévue sur les deux faces du support.

6. Implant selon l'une quelconque des revendications 1 à 4, **caractérisé en ce que** le support présente une couche adhésive sur une face et de préférence une couche anti-adhésive sur l'autre face.

7. Implant selon la revendication 6, **caractérisé en ce que** la couche anti-adhésive présente une surface fermée et en particulier lisse.

8. Implant selon l'une quelconque des revendications précédentes, **caractérisé en ce que** la couche adhésive est conçue sous la forme d'une couche ouverte et en particulier absorbante.

9. Implant selon l'une quelconque des revendications précédentes, **caractérisé en ce que** la couche adhésive est hydrophile et en particulier capable d'absorber des liquides aqueux par gonflage.

10. Implant selon l'une quelconque des revendications précédentes, **caractérisé en ce que** la couche adhésive se présente sous la forme d'un non-tissé, en particulier tridimensionnel.

11. Implant selon l'une quelconque des revendications 1 à 9, **caractérisé en ce que** la couche adhésive se présente sous la forme d'une mousse à alvéoles ouvertes.

12. Implant selon l'une quelconque des revendications 2 à 11, **caractérisé en ce que** le polymère portant des groupes aldéhyde est hydrosoluble.

13. Implant selon l'une quelconque des revendications 2 à 12, **caractérisé en ce que** le polymère portant des groupes aldéhyde est un polysaccharide oxydé, en particulier bio-absorbable.

14. Implant selon la revendication 13, **caractérisé en ce que** le polysaccharide oxydé est l'un parmi le groupe constitué de l'amidon, de la cellulose, de la gélose, du dextrane-aldéhyde, de l'acide hyaluronique, de l'acide alginique, du sulfate de chondroïtine et de préférence du dextrane-polyaldéhyde.

15. Implant selon la revendication 14, **caractérisé en ce que** la proportion de motifs glucose oxydés en aldéhyde dans le dextrane-polyaldéhyde est d'au moins 20 %, de préférence de 35 à 100 %, en particulier de 50 à 85 %.

16. Implant selon l'une quelconque des revendications 2 à 12, **caractérisé en ce que** le polymère portant des groupes aldéhyde est un polyéthylèneglycol en particulier ramifié, renfermant au moins trois groupes aldéhyde terminaux.

17. Implant selon l'une quelconque des revendications 2 à 12, **caractérisé en ce que** le polymère portant des groupes aldéhyde est un alcool polyvinylique en particulier ramifié, renfermant au moins trois groupes aldéhyde terminaux.

18. Implant selon l'une quelconque des revendications 2 à 17, **caractérisé en ce que** l'au moins un polymère portant des groupes aldéhyde est partiellement réticulé.

19. Implant selon l'une quelconque des revendications précédentes, **caractérisé en ce que** la couche adhésive présente une surface structurée sur sa face externe.

20. Implant selon l'une quelconque des revendications précédentes, **caractérisé en ce que** le support plat est poreux et souple, et est en particulier formé d'une matière textile.

21. Implant selon l'une quelconque des revendications précédentes, **caractérisé en ce que** le support, en particulier le support textile, est au moins partiellement, en particulier totalement résorbable.

22. Implant selon l'une quelconque des revendications précédentes, **caractérisé en ce qu'**une face du support est pourvu d'au moins une couche anti-adhésive qui est de préférence résorbable.

23. Implant selon la revendication 21, **caractérisé en ce que** la couche anti-adhésive contient de l'alcool polyvinylique et/ou de la carboxyméthylcellulose, et est en particulier constituée d'alcool polyvinylique.

24. Implant selon l'une quelconque des revendications précédentes, **caractérisé en ce qu'**il est conçu sous la forme d'un treillis pour hernie qui présente la couche adhésive sur la face qui est destinée à être placée sur la paroi abdominale, et **en ce que** l'autre face du treillis pour hernie présente de préférence au moins une couche qui est conçue en tant que couche anti-adhésive et empêche une adhésion du tissu corporel au treillis.

25. Implant selon l'une quelconque des revendications 1 à 22, **caractérisé en ce qu'**il est conçu en tant qu'emplâtre (patch) qui présente la couche adhésive sur au moins une face.

26. Implant selon l'une quelconque des revendications 1 à 22, **caractérisé en ce qu'**il se présente sous la forme d'une section de tube qui est conçue pour relier des organes creux en forme de tube ou tuyau.
